# EUROPEAN PATENT SPECIFICATION

(11) **EP 1 259 228 B1**
(45) Date of publication and mention of the grant of the patent: **06.01.2010**
(21) Application number: 01907916.9
(22) Date of filing: 27.02.2001
(51) Int. Cl.: A61K 9/16, A61K 9/72

(54) **THERAPEUTIC COMPOSITIONS FOR PULMONARY DELIVERY**
THERAPEUTISCHE ZUSAMMENSETZUNGEN ZUR PULMONALEN VERABREICHUNG
COMPOSITIONS THERAPEUTIQUES POUR ADMINISTRATION PULMONAIRE

(30) Priority: 29.02.2000 GB 0004827
(43) Date of publication of application: 27.11.2002
(73) Proprietor: Quadrant Drug Delivery Limited, Nottingham, NG11 6JS (GB)
(72) Inventor: MARTYN, Glen P., Quadrant Healthcare (UK) Limited, Ruddington, Nottingham NG11 6JS (GB)
(74) Representative: Jappy, John William Graham
(86) International application number: PCT/GB2001/000834
(87) International publication number: WO 2001/064188

(56) References cited:
- EP-A- 0 517 565
- EP-A- 0 611 567
- WO-A-00/59476
- WO-A-01/19345
- WO-A-90/13285
- WO-A-97/35562
- WO-A-98/43664
- US-A- 5 922 253

## Description

### Field of the Invention

The present invention relates to the manufacture of particles that may be used to deliver a therapeutic agent via the lung, and compositions thereof.

### Background to the Invention

The delivery of therapeutic agents to a patient via the lung is now well established. Compositions for pulmonary delivery are usually aerosolised in an inhaler device, activated by inhalation from the patient. In order to deposit the active agent effectively within the lung, the inhalable compositions should exhibit specific properties. For example, it is usually necessary to have inhalable particles of a small aerodynamic size and shape, and particle diameter of typically less than 20 µm, and preferably less than 10 µm. This is to ensure that the particles are able to penetrate deep within the lung. The compositions are usually in the form of powders which exhibit minimal electrostatic activity, low hygroscopicity, and have good flow properties. It is also preferable that the therapeutic is in a sustained release formulation to maintain a constant release of the therapeutic over time, thus sustaining the therapeutic effect. For this reason, the therapeutic is often contained within a carrier material which exhibits these release properties.

Many different carriers have been proposed for use in pulmonary delivery. Carbohydrates and polysaccharides have long been considered good carrier materials as they can be formulated easily into stable compositions, and have good release properties. WO-A-98/43664 discloses the use of hyaluronic acid as a carrier material. Hyaluronic acid is stated to have good sustained-release properties. Although the main focus of the description is with respect to injection formulations, there is mention of an aerosol formulation for delivery via the nose or bronchi mucus membrane. The manufacture of the compositions is shown to be by spray-drying.

Although the compositions may have suitable properties for pulmonary delivery, there is still a need for improved formulations to promote delivery of therapeutic agents delivered via the lung.

### Summary of the Invention

The present invention is based on the surprising finding that the process of spray-freeze-drying can be used to produce microparticles which exhibit beneficial properties for pulmonary delivery.

According to one aspect of the invention, microparticles are obtainable by spray-freeze-drying a solution or dispersion comprising a water-soluble, matrix-forming polymer and a therapeutic agent wherein the polymer is a polysaccharide with a molecular weight greater than 500kDa and wherein the therapeutic agent is dispersed within the polymer matrix. Hyaluronic acid, or an inorganic salt thereof, is a particularly preferred polymer.

According to a second aspect of the invention, a composition for pulmonary delivery comprises microparticles as defined above, and a carrier material.

According to a third aspect, a device for delivery of a therapeutic agent via pulmonary inhalation comprises a microparticle as defined above.

According to a fourth aspect of the invention, a process for the preparation of microparticles for pulmonary delivery comprises spray-freeze-drying a solution or dispersion comprising a therapeutic agent and a water-soluble, matrix-forming polymer.

Spray-freeze-drying the matrix-forming polymer and therapeutic agent results in microparticles that are lighter than conventional spray-dried particles, with good porous characteristics and which are therefore better able to achieve deep lung deposition. High molecular weight polymers suitable for use in the invention also exhibit good mucoadhesive properties, and are therefore particularly suitable for pulmonary delivery. In addition, the polymers used to produce the microparticles have good controlled release properties, and are therefore more beneficial than conventional low molecular weight sugars for delivery via the pulmonary route. The spray-freeze-drying process results in improved recovery of the product compared to that recovered from conventional spray-drying methods.

### Description of the Invention

The present invention makes use of spray-freeze-drying technology to manufacture novel microparticles particularly suited to pulmonary delivery. The process of spray-freeze-drying involves the atomisation of a solution or dispersion of the matrix-forming polymer and therapeutic agent, and then directing the resulting droplets into a liquified gas, typically liquid nitrogen. The droplets freeze on contact with the liquified gas and may then be dried using a freeze-drying step to remove residual moisture. The resulting microparticles comprise a therapeutic agent dispersed within the polymer matrix.

The apparatus and process conditions used to produce the initial droplets will be apparent to the skilled person. Feed concentrations, pump rates, atomisation pressures and nozzle types can all be selected based on conventional process conditions, and then optimised according to feedstock concentration and viscosity.

The size of the microparticles will be determined in part by the atomisation used in the spray-freeze-drying process. The atomisation/spraying stage may make use of a conventional atomisation process, e.g. pressure or two fluid nozzles, or may utilise an ultrasonic atomisation process (Maa et al., Pharmaceutical Research, 1999; 16(2)). The microparticles will usually have a mean aerodynamic particle diameter size ranging from 0.1 to 40 µm, preferably from 0.1 to 10 µm, and most preferably from 0.1 to 5 µm. This may be measured using a aerosizer (TSI Instruments) as will be appreciated by the skilled person.

The drying process may be carried out using conventional freeze-drying apparatus. Drying will usually be carried out to achieve a residual moisture content of the microparticles of less than 10% by weight, preferably less than 5% by weight and most preferably less than 3% by weight.

The matrix-forming polymer should be water-soluble, i.e. hydrophilic. High molecular weight polysaccharides are a preferred embodiment, as are gums and cellulose ethers. Hyaluronic acid is a particularly preferred polymer as it exhibits good mucoadhesive properties, is biocompatible and biodegradable, and is also able to avoid phagocytic uptake. Other suitable polymer materials include hydrogels, alginic acid, pectins, agarose, and polyvinylpyrrolidone.

As used herein, the reference to "matrix-forming polymer" is intended to mean that the polymer forms a stable, rigid, structure capable of retaining molecules that may be dispersed therein. Polymers are typically made up of multiple repeating monomer units, typically greater than three monomer units. In the context of the present invention, high molecular weight polymers are greater than 250 kDa, preferably greater than 500 kDa, more preferably greater than 1000 kDa and most preferably greater than 1500 kDa. High molecular weight, hydrophilic polymers are particularly suitable for pulmonary delivery as they offer beneficial controlled release properties, which ensures that a therapeutic agent, dispersed within the polymer, can be administered in a controlled manner over time. This is different from the use of conventional low molecular weight sugars, e.g. monosaccharides and disaccharides, which may be rapidly soluble on administration.

The polymer should be physiologically acceptable. It is preferred if the polymer is capable of stabilising the therapeutic agent during the preparation of the microparticles and storage. This is particularly important when the therapeutic agent is a protein or peptide, which may be relatively labile.

The amount of polymer in the initial feedstock can be determined by the skilled person, depending on the properties required. Dilute concentrations are preferred, preferably 0.01 % w/v to 20% w/v, more preferably 0.01 % w/v to 1 % w/v, most preferably 0.1% w/v to 0.5% w/v.

Any suitable therapeutic agent may be used in the present invention, as will be appreciated by the skilled person. Therapeutic agents which may be used include, for example, proteins, peptides, nucleic acids and small organic molecules. Anti-inflammatory compounds are preferred, as is insulin in its hexameric or monomeric form. The reference to therapeutic agents is intended to also include prophylactic agents, including vaccines in the form of proteins or polypeptides, or attenuated microorganisms. Pharmaceutical agents that are particularly suitable for administration via the pulmonary route are preferred. In particular, antiallergics, bronchodilators, analgesics, antibiotics, antihistamines, antiinflammatories, steroids, cytokines, cardiovascular agents and immunoactive agents.

It will be appreciated by the skilled person that the therapeutic agents are to be formulated in physiologically effective amounts. That is, when delivered in a unit dosage form, there should be a sufficient amount of the therapeutic to achieve the desired response. As the microparticles of the invention are intended primarily for delivery as dry powders in an inhalation device, it will be appreciated that a unit dose comprises a predefined amount of microparticles delivered to the patient in one inspiratory effort. In a preferred embodiment, the microparticles are prepared as single unit dosage forms for inclusion in dry powder inhalers. In this embodiment, a single unit dose will be approximately 1 to 15 mg, preferably between 5 to 10 mg.

The amount of therapeutic agent present in each microparticle will be determined on the basis of the level of biological activity exhibited by the therapeutic agent. If the therapeutic agent has high activity, then there may be as little as 0.001% w/w of the agent with respect to the polymer material. Usually the microparticles will comprise greater than 5%, 20%, 30% or even 40% w/w of the therapeutic agent. The amounts can be controlled simply by regulating the concentration of the agent in solution with the polymer prior to the spraying step.

The composition to be spray-freeze-dried may also comprise other components, e.g. carbohydrates or other glass-forming substances as stabilisers or excipients. Additional components may be desirable to modify the characteristics of the microparticles. For example, it may be desirable to add further components to improve the particle rigidity or release profile. Surfactants may be used in the microparticle formulations to improve the flowability of the microparticles or to improve dispersion stability or to aid in the preparation of the initial feedstock. Examples of suitable surfactants include long-chain phospholipids, e.g. phosphatidylcholines, phosphatidylglycerols and polyethylene glycol. Other suitable surfactants include sorbitan esters, sorbitan monooleate and glycerol esters. In order to use the surfactants, it may be necessary to utilise a co-solvent system, e.g. aqueous organic solvents. Buffers and salts may also be included. Other suitable excipients will be apparent to the skilled person.

The microparticles are intended primarily for delivery via inhalation. The preferred delivery system is a dry powder inhaler (DPI), which relies entirely on the patient's inspiratory efforts to introduce the microparticles in a dry powder form into the lungs. However, alternative inhalation devices may also be used. For example, the microparticles may be formulated for delivery using a metered dose inhaler (MDI), which usually requires a high vapour pressure propellant to force the microparticles into the respiratory tract. Nebulisers are also envisaged. These require aerosol formulations, which will be apparent to the skilled person.

In the context of dry powder inhalers, the microparticles may be formulated in compositions further comprising bulk carrier particles, which aid delivery. Suitable carrier particles are known, and include crystalline lactose particles, of a size typically in the range of from 30 to 300 µm, more usually 50 µm to 250 µm. However, as the microparticles of the invention exhibit improved aerodynamic properties, it is envisaged that carrier particles will not be required. This has the added benefit of allowing more microparticles to be prepared in a single dosage form, which ensures more flexibility in the dosage regimen to be adopted for any particular therapeutic agent.

The following Examples illustrate the invention.

### Example 1

Aqueous solutions of hyaluronic acid and insulin were prepared in a 1:1 ratio. The atomisation stage was carried out using an two-fluid nozzle air atomiser. The solution was sprayed at room temperature into a round metal container which contained stirred liquid nitrogen. The liquid feed-rate was 3.5 mls/minute through the nozzle. The sprayed particles froze immediately on contact with the liquid nitrogen. After the spraying process had been completed, the liquid nitrogen was transferred to a lyophiliser (FTS) which had been pre-chilled to -50° C. Freeze-drying occurred with a vacuum of 0.1 m bar and primary drying occurred at a shelf temperature of -20°C for 30 hours. Secondary drying was carried out at 20°C for 15 hours.

Respirable powders were provided.

### Example 2

A solution comprising 0.2% w/v hydroxy propyl cellulose (Klucel HXF) and 0.1 % w/v human serum albumin (HSA) was dispensed at room temperature into a liquid nitrogen bath using an IVEK model AAA pump with the droplet volume adjusted to 5 µl. The resulting frozen spheres were transferred to a pre-chilled drying chamber at -50°C. A vacuum of 0.1 mbar was then applied for 30 hours, then raised to 20°C for a further 15 hours.

Respirable powders were obtained.

### Example 3

A warm solution comprising 0.2% w/v agarose and 0.1 % w/v HSA was dispensed into a liquid nitrogen bath using a Schlick pressure nozzle, with a 0.2 mm bore. The resulting frozen droplets were transferred to a pre-chilled drying chamber at -50°C. A vacuum of 0.1 mbar was then applied for 30 hours, then raised to 20°C for a further 15 hours.

Respirable, free-flowing powders were obtained which dissolved slowly.

## Claims

1. Microparticles obtainable by spray-freeze-drying a solution comprising a water-soluble, matrix-forming polymer and a therapeutic agent wherein the polymer is a polysaccharide with a molecular weight greater than 500KDa and wherein the therapeutic agent is dispersed within the polymer matrix

2. Microparticles according to claim 1 wherein the polymer is hyaluronic acid, or an inorganic salt thereof

3. Microparticles according to claim 1 or claim 2, wherein the microparticles have a mean aerodynamic particle diameter size from 0 1 µm to 40 µm.

4. Microparticles according to any preceding claim, wherein the therapeutic agent is a nucleic acid.

5. Microparticles according to any of claims 1 to 3, wherein the therapeutic agent is a protein or peptide.

6. Microparticles according to claim 5, wherein the therapeutic agent is insulin.

7. Microparticles according to any preceding claim, further comprising a carbohydrate.

8. Microparticles according to any preceding claim, further comprising a surfactant.

9. A composition suitable for pulmonary delivery, comprising microparticles according to any preceding claim, and a carrier particle.

10. A composition according to claim 9, wherein the carrier particle is from 30 um to 300 µm in diameter.

11. A composition according to claim 9 or claim 10, wherein the carrier particle is lactose.

12. A device for delivery of a therapeutic agent via pulmonary inhalation, wherein the device incorporates a microparticle according to any of claims 1 to 8 or a composition according to any of claims 9 to 11

13. Use of microparticles according to any of claims 1 to 8, in the manufacture of a composition for pulmonary administration for the treatment of disease.

14. A process for the production of microparticles according to claim 1 suitable for pulmonry administration, comprising spray-freeze-drying a solution or dispersion comprising therapeutic agent and a hydrophilic matrix-forming polymer.

15. A process according to claim 14, wherein the spray-freeze-drying is carried out under conditions to produce microparticles of from 1 µm to 20 µm in diameter.

16. A process according to claim 14 or claim 15, wherein the polymer is hyaluronic acid, or an inorganic salt thereof.

## Patentansprüche

1. Mikropartikel, erhältlich durch Sprüh-Gefriertrocknen einer Lösung, die ein wasserlösliches, Matrix-bildendes Polymer und ein Therapeutikum umfasst, worin das Polymer ein Polysaccharid mit einem Molekulargewicht von größer als 500 kDa darstellt und worin das Therapeutikum in der polymeren Matrix dispergiert ist.

2. Mikropartikel nach Anspruch 1, worin das Polymer für Hyaluronsäure oder ein anorganisches Salz davon steht.

3. Mikropartikel nach Anspruch 1 oder 2, worin die Mikropartikel eine mittlere aerodynamische Partikeldurchmessergröße von 0,1 µm bis 40 µm aufweisen.

4. Mikropartikel nach einem der vorangehenden Ansprüche, worin das Therapeutikum eine Nukleinsäure darstellt.

5. Mikropartikel nach einem der Ansprüche 1 bis 3, worin das Therapeutikum ein Protein oder Peptid darstellt.

6. Mikropartikel nach Anspruch 5, worin das Therapeutikum Insulin darstellt.

7. Mikropartikel nach einem der vorangehenden Ansprüche, die ferner ein Kohlenhydrat umfassen.

8. Mikropartikel nach einem der vorangehenden Ansprüche, die ferner ein Tensid umfassen.

9. Zusammensetzung, die zur pulmonalen Abgabe geeignet ist, welche Mikropartikel nach einem der vorangehenden Ansprüche und ein Trägerpartikel umfasst.

10. Zusammensetzung nach Anspruch 9, worin das Trägerpartikel von 30 µm bis 300 µm im Durchmesser ist.

11. Zusammensetzung nach Anspruch 9 oder 10, worin das Trägerpartikel für Lactose steht.

12. Vorrichtung zur Abgabe eines Therapeutikums über die pulmonale Inhalation, worin die Vorrichtung ein Mikropartikel nach einem der Ansprüche 1 bis 8 oder eine Zusammensetzung nach einem der Ansprüche 9 bis 11 inkorporiert.

13. Verwendung von Mikropartikeln nach einem der Ansprüche 1 bis 8, in der Herstellung einer Zusammensetzung zur pulmonalen Verabreichung zur Behandlung einer Krankheit.

14. Verfahren zur Herstellung von Mikropartikeln nach Anspruch 1, die zur pulmonalen Verabreichung geeignet sind, umfassend die Sprüh-Gefriertrocknung einer Lösung oder Dispersion, die das Therapeutikum und ein hydrophiles Matrix-bildendes Polymer umfasst.

15. Verfahren nach Anspruch 14, worin die Sprüh-Gefriertrocknung unter Bedingungen zur Herstellung von Mikropartikeln von 1 µm bis 20 µm im Durchmesser durchgeführt wird.

16. Verfahren nach Anspruch 14 oder 15, worin das Polymer Hyaluronsäure oder ein anorganisches Salz davon darstellt.

## Revendications

1. Microparticules pouvant être obtenues par pulvérisation-cryodessiccation d'une solution comprenant un polymère formateur de matrice hydrosoluble et un agent thérapeutique, dans laquelle le polymère est un polysaccharide de poids moléculaire supérieur à 500 kDA et dans laquelle l'agent thérapeutique est dispersé dans la matrice polymérique.

2. Microparticules selon la revendication 1, dans laquelle le polymère est l'acide hyaluronique, ou un sel inorganique de celui-ci.

3. Microparticules selon la revendication 1 ou 2, dans laquelle les microparticules ont un diamètre de particule aérodynamique moyen compris entre 0,1 µm et 40 µm.

4. Microparticules selon l'une quelconque des revendications précédentes, dans laquelle l'agent thérapeutique est l'acide nucléique.

5. Microparticules selon l'une quelconque des revendications 1 à 3, dans laquelle l'agent thérapeutique est une protéine ou un peptide.

6. Microparticules selon la revendication 5, dans laquelle l'agent thérapeutique est l'insuline.

7. Microparticules selon l'une quelconque des revendications précédentes, comprenant, en outre, un glucide.

8. Microparticules selon l'une quelconque des revendications précédentes comprenant, en outre, un agent surfactant.

9. Composition convenant à l'administration par voie pulmonaire, comprenant des microparticules selon l'une quelconque des revendications précédentes, et une particule vectrice.

10. Composition selon la revendication 9, dans laquelle la particule vectrice a un diamètre compris entre 30 µm et 300 µm.

11. Composition selon la revendication 9 ou 10, dans laquelle la particule vectrice est le lactose.

12. Dispositif pour l'administration d'un agent thérapeutique par inhalation pulmonaire, dans lequel le dispositif incorpore une microparticule selon l'une quelconque des revendications 1 à 8 ou une composition selon l'une quelconque des revendications 9 à 11.

13. Utilisation de microparticules selon l'une quelconque des revendications 1 à 8, dans la fabrication d'une composition pour administration par voie pulmonaire pour le traitement de maladies.

14. Processus pour la production de microparticules selon la revendication 1 et convenants à l'administration par voie pulmonaire, comprenant les étapes de pulvérisation-cryodessiccation d'une solution ou dispersion comprenant un agent thérapeutique et un polymère formateur de matrice hydrophile.

15. Processus selon la revendication 14, dans lequel la pulvérisation-cryodessiccation est effectuée sous des conditions produisant des microparticules d'un diamètre compris entre 1 µm et 20 µm.

16. Processus selon la revendication 14 ou 15, dans lequel le polymère est l'acide hyaluronique, ou un sel inorganique de ce dernier.
